Europäisches Patentamt

(19) European Patent Office

Office européen des brevets

(11) **EP 0 768 061 A1**

(12) **EUROPEAN PATENT APPLICATION**

(43) Date of publication:
16.04.1997 Bulletin 1997/16

(51) Int. Cl.$^6$: **A61B 6/00**

(21) Application number: 96117335.8

(22) Date of filing: 12.11.1993

(84) Designated Contracting States:
**AT BE CH DE DK ES FR GB IT LI LU NL SE**

(30) Priority: 16.11.1992 US 976797

(62) Document number(s) of the earlier application(s) in accordance with Art. 76 EPC:
**94901413.8 / 0 682 497**

(71) Applicant: **LUNAR CORPORATION**
**Madison Wisconsin 53713 (US)**

(72) Inventors:
• **Mazess, Richard B.**
**Madison, Wisconsin 53711 (US)**

• Hanson, James A.
**Madison, Wisconsin 53705 (US)**
• Deluhery, James G.
**Madison, Wisconsin 53713 (US)**

(74) Representative: **Ford, Michael Frederick et al**
**MEWBURN ELLIS**
**York House**
**23 Kingsway**
**London WC2B 6HP (GB)**

Remarks:
This application was filed on 29 - 10 - 1996 as a divisional application to the application mentioned under INID code 62.

(54) **X-ray scanning of bone**

(57) The invention relates to methods of using a bone densitometer to scan bone, notably vertebrae. The bone is scanned while maintaining the X-ray source (44) and detector (50) at a first angle, and a two dimensional array (78) of pixels (76) is obtained. This is analysed electronically and used to adjust the position of the detector (50) relative to the patient during a second scan at a different angle. Electronic analysis of the data is used to eliminate the effect of osteophytes on the bone density image.

FIG. 13

EP 0 768 061 A1

**Description**

Field of the Invention

The present invention relates generally to radiographic instruments and their use and more particularly to a versatile instrument for either radiographic scanning or tomographic scanning of a patient for evaluation of bone density and bone morphology.

Background of the Invention

Scanning Radiography

Scanning radiographic equipment differs from conventional radiography in that it employs a narrowly collimated beam of radiation, typically x-rays formed into a fan or pencil beam, rather than a broad area cone beam. The small beam size used in scanning radiographic equipment allows replacement of an image forming sheet of radiographic film, used with conventional radiographic equipment, with a small area electronic detector element or array of such elements.

The detector elements receiving the transmitted radiation produce electrical signals which may be converted to digital values by an analog to digital converter for the later development of an image or for other processing by computer equipment. The ability to quantify the measurement of the transmitted radiation, implicit in the digitization by the analog to digital converter, allows not only the formation of a radiographic "attenuation" image but also the mathematical analysis of the composition of the attenuating material by dual energy techniques. See generally, "Generalized Image Combinations in Dual KVP Digital Radiography", by Lehmann et al. Med. Phys. 8(5) Sept/Oct 1981.

Such dual energy techniques quantitatively compare the attenuation of radiation at two energies to distinguish, for example, between bone and soft tissue. Dual energy techniques allow the measure of bone mass, such measurement being important in the treatment of osteoporosis and other bone diseases.

The limited area of the beam of radiation used in scanning radiographic systems requires that the beam be moved over an area, if a conventional image is to be formed. Typically, the pencil or fan beam will be scanned in a raster pattern over the area to be measured, each line of the scan separated by the width of the pencil or fan beam, with the directions of scanning being generally perpendicular to the direction of the radiation.

Images formed by a scanning radiographic system are potentially more accurate than those produced by a typical broad beam radiograph system. This accuracy arises from the limited divergence of the rays of the pencil or fan beam from the principal axis of the radiation, as compared to a broad area cone beam. This narrow collimation of the pencil or fan beam reduces "parallax" in the projected image, potentially providing extremely accurate morphological measurements of certain structures such as the vertebrae in the spine. Such morphological measurements are used to evaluate various dimensions of a vertebra to detect crushing or other deformation that are one element of certain bone diseases such as osteoporosis. See e.g. Minne et al., "A Newly Developed Spine Deformity Index (SDI) to Quantitate Vertebral Crush Factors in Patients with Osteoporosis," Bone and Mineral, 3:335-349 (1988); J. C. Gallagher et al, "Vertebral Morphometry: Normative Data," Bone and Mineral, 4:189-196 (1988); Hedlund et al, "Vertebral Morphometry in Diagnosis of Spinal Fractures," Bone and Mineral, 5:59-67 (1988); and Hedlund et al, "Change in Vertebral Shape in Spinal Osteoporosis," Calcified Tissue International, 44:168-172 (1989). Automatic techniques for morphological measurements of bone are described in U.S. patent application serial number 07/944,626 filed September 14, 1992, entitled: "Method for Analyzing Vertebral Morphology Using Digital Radiography" and published as US-A-5,228,068 on July 13, 1993 and hereby incorporated by reference.

In order to make accurate morphological measurements and to provide clinically valuable dual energy measurements of a variety of body structures, the radiation source and detector should be easily positioned at different angles about the patient. Further, at each such angle, the radiation source and detector must have the necessary clearance from the patient to perform the required scanning.

Computed Tomography

In a computed tomography system ("CT system") the radiation source and detector are be rotated on a gantry about the patient and within an imaging plane so that measurements of the imaged object at different angles may be obtained. At each angle, a projection is acquired comprised of the attenuation measurements of the radiation along different rays or beams through the patient. The projections at each of these different angles together form a tomographic projection set. Normally projections must be acquired over a range of no less than an 180° when the beams of each projection are parallel. If less than a full projection set is acquired, or less than the full width of the patient measured at each angle, undesirable image artifacts will appear in the reconstructed image.

In fan beam CT systems the radiation from the radiation source is formed into a fan within the imaging plane so that multiple beams may be measured simultaneously at each angle about the imaging plane for the full width of the patient. Such fan beam systems, in contrast to systems that scan with a pencil beam at each location, can significantly increase the speed at which the projection set is acquired, but because the detector must be wide enough to capture the entire fan of beams at one time.

The acquired tomographic projection set is typically stored in numerical form for computer processing to "reconstruct" a slice image according reconstruction algorithms known in the art. The reconstructed slice images may be displayed on a conventional CRT tube or may be converted to a film record by means of a computer controlled camera.

Many of the components used to perform scanning radiography and computer tomography are similar, especially where the scanning radiography is undertaken with dual energy measurements and thus employs computer reconstruction. This makes a combined scanning radiographic system and tomographic system attractive from a cost viewpoint, particularly for institutions where a full capacity CT scanner could not be justified. Nevertheless, the design of such a combined system presents significant. challenges.

First, the need to acquire a full projection set of projections about the patient for CT reconstruction, normally requires a large and complex rotating gantry for supporting the radiation source and detector. The gantry mechanism is costly and may be impractical for smaller institutions and many diagnostic procedures. In this respect, it is noted that most current CT scanners can, in fact, perform scanning radiography, but are rarely used for this purpose because their cost is not justified by such procedures.

Second, for practical CT scanning speeds, large multi-element detectors and high speed data processors are needed to acquire and process the large amounts of projection data. These elements contribute to the high cost of CT capabilities.

In one aspect the present invention provides a method of making measurements of vertebrae of a patient's spine with a densitometer having an opposed radiation source and detector, the source and detector moving to a first and second angle about the spine and at each angle scanning to obtain a two dimensional array of pixels representing the attenuation of radiation at corresponding locations through the patient, comprising the steps of:

positioning the source and detector at the first angle and scanning the vertebrae along a first axis to obtain the first array of pixels;

analyzing the first array of pixels to determine center of bone mass values at a plurality of points along an axis of the array corresponding to the first axis;

positioning the source and detector at a second angle substantially perpendicular to the first angle;

scanning along the first axis of the patient to obtain the second array of pixels;

adjusting the position of the detector with respect to the patient during the scanning at the second angle in accordance with the determined center of bone mass values of the first array of pixels.

In this method, the invention employs two scans at different angles with the first scan being used to correct the second scan which may then be used for morphological measurements.

The source and detector may be positioned at two substantially perpendicular angles and a scan of the patient obtained at these angles to produce the first and second array of pixels, each pixel representing bone mass at a corresponding location in the patient. During the scanning of the patient at the second angle along this principle scanning direction, the position of the detector with respect to the patient is adjusted in accordance with the determined center of bone mass values. The detector may be adjusted to maintain an essentially constant distance with respect to the center of mass of the patient, indicated by the center of mass values, and hence a constant magnification of the spine. Or the angle of the radiation received by the detector may be adjusted to remain substantially perpendicular to the spine at the point of imaging.

Adjustment of positioning of the detector during the second scan allows the fidelity of the generated image to be improved for dimension measuring purposes.

It is another object of the invention to remove the effects of osteophytes, i.e. bone elements having density harder than normal bone, from the calculation of bone density and other bone measurements. The closeness of the osteophytes to the density values of normal bone renders simple thresholds techniques inaccurate.

A histogram of selected pixels acquired during the scan may be reviewed to compile a multiple-mode density histogram providing number of pixels as a function of pixel density value. From this histogram, a soft tissue average and hard tissue average may be determined corresponding to average density values of soft and hard tissue, such as bone, respectively. A threshold above the hard tissue average based on the difference between the hard tissue average and the soft tissue average is generated and the hard tissue average is recomputed ignoring pixels above the threshold. This process is repeated until the change in the computed hard tissue average is below a predetermined threshold and that hard tissue average is designated as the density of the region of interest.

The methods of the present invention can be performed using a scanning radiographic system employing a compact and cost-effective C-arm gantry and a small detector array which is capable of practical use as a tomography machine. A small detector reduces the need for high speed processing equipment. Dual energy measurements are used to reduce artifacts caused by the small detector's acquiring less than a full beam width of data.

Specifically, the x-ray apparatus may include a track for moving a pallet with respect to a patient along a first and second perpendicular axis. A collar attached to the pallet holds a C-arm which may slide through the collar so that its ends rotate to one of a plurality of angles about the patient. A radiation source and a detector are affixed to the ends of the C-arm to provide energy attenuation measurements along an axis between those ends at the plurality of angles, such measurements being received by an electronic computer. The electronic computer controls the C-arm, the radiation source and the detector according to a stored program so as to rotate the C-arm through the angles and to store the attenuation measurements at those angles. The computer then reconstructs the stored attenuation measurements into a tomographic image. The C-arm may include an arcuate arm portion received in the collar and a carriage attached to the arm for sliding inward from one end of the arm to a plurality of distances. One of the radiation source and radiation detector may be attached to the carriage for movement with the carriage. This movement provides an additional angular range of the C-arm.

In addition or alternatively, a sleeve may be attached to one end of the arcuate arm holding a support beam having a mounting end projecting from the arcuate arm so as to extend a plurality of distances from the end of the arcuate arm with the sliding of the beam in the sleeve. One of the radiation source and radiation detector may be attached to the mounting end to move with the sliding of the beam. Again, an increase in effective angular range of the C-arm is thereby produced.

The electronic computer may estimate from the attenuation measurements taken at the plurality of positions, further attenuation measurements that complete a tomographic projection set. These projection measurements reduce the angular motion required of the C-arm.

The detector may receive a fan of radiation extending less than the full width of an average patient positioned between the radiation source and the detector, and may produce separate attenuation measurements indicating attenuation at two energy levels. The electronic computer may process the attenuation measurements to produce attenuation measurements dependent on the attenuation of a single material only. In this way it is possible to limit the amount of data that needs to be processed when imaging a compact structure of distinguishable material within the patient, such as a vertebra. Dual energy measurement allows isolation of the compact structure from the surrounding tissue eliminating partial volume image artifacts in the CT reconstruction, such artifacts resulting from soft tissue which is in some projections and not in other projections.

The radiation source may produce a fan beam of rays of radiation diverging along a fan beam plane and the detector may be a linear array of detector elements, each element measuring the intensity of radiation along one ray of the fan beam. The fan beam and detector array may be mounted to the C-arm so that the fan beam and the detector array may rotate about a fan beam axis connecting the radiation source and the detector array.

The fact that both the radiation source and the detector are rotatable about a line between the two permits the scanning direction to be changed with the orientation of the fan beam adjusted so that the direction of scanning remains perpendicular to the fan beam plane. The ability to adjust the direction of scanning reduces the effect of patient motion on the resultant data by allowing the selection of a scanning pattern where adjacent points in space are scanned at proximate times.

The detector array for the fan beam may be rotated independently of the radiation source so as to reduce its swept volume during motion of the gantry.

Rotation of the detector array to its orientation of least swept volume can allow the detector array to pass through the reduced width area of the table during movement of the gantry to a new position without translation of the gantry.

In one embodiment, a support beam having a mounting end may be attached to the first end of the C-arm with a sleeve so that it may slide away from the first end to a plurality of distances. Either the radiation source or detector may be attached to the mounting end of the support beam. In addition, the other of the source or detector may be attached to a carriage that slides on the inner radius of the C-arm in from the second end.

A preferred embodiment of the invention will now be described by way of example only, with reference to the accompanying drawings.

Brief Description of the Drawings

Fig. 1 is a perspective view of the present invention showing a C-arm, having an x-ray source and a detector, and a table positioned for access by the patient and a controlling computer;

Figs. 2(a) and 2(b) are plan and elevation views of the table of Fig. 1 showing the hourglass shape of the table and two possible scanning patterns that may be employed by the present invention;

Fig. 3(a) is a cross-section of one support for the table of Fig. 1 taken along line 3(a)-3(a) in Fig. 1 showing the upward curvature of the table surface and the mechanism for elevating and lowering the table;

Fig. 3(b) is a cross-section of the support of Fig. 3(a) taken along line 3(b)-3(b) of Fig 3(a);

Fig. 4 is an exaggerated depiction of the table in conjunction with a radiation source and detector showing the effect of table height on the divergence of the rays of the illuminating radiation as is related to problems of parallax;

Fig. 5 is a cross section of the gantry taken along lines 5-5 of Fig. 1 showing an internal sleeve and support beam for holding the radiation source at a variety of distances from the end of the gantry C-arm and the different radiuses of motion produced by motion of the C-arm and motion of the sliding support beam;

Fig. 6 is a view in elevation of the densitometer of Fig. 1 taken along the longitudinal axis showing displacement of the center of rotation of the C-arm and the rotation of the C-arm for proper imaging of a femur of the patient without inward rotation of the patient's leg;

Fig. 7 is a plan view of a detail of the table of Fig. 1 showing interference between the table and the detector array when the detector array is in a first position, in phantom, and clearance when the detector array is in the second position;

Fig. 8 is a exploded schematic view of the radiation source and a perspective view of the detector showing rotation of the fan beam by movement of a collimator and corresponding motion of the detector array;

Fig. 9 is a perspective cutaway view of the supporting mechanism for the C-arm of Fig. 1 showing rotation of the C-arm with respect to the gantry pallet and the x and y translation of the pallet;

Fig. 10 is a detailed of a schematic, anterior/posterior view of the spine of a patient showing the resolution of the vertebrae into pixels for analysis;

Fig. 11 is a flow chart illustrating the steps of determining the curvature of the spine of Fig. 10 in the lateral plane of the vertebrae of the patient such as may occur in sclerosis;

Fig. 12 is a schematic, plan view showing the movement of the x-ray source and the detector of Fig. 1 to follow the curvature of the spine in lateral imaging, per the density measuring view of Fig. 10, according to a first embodiment;

Fig. 13 is a schematic, plan view showing the movement of the x-ray source and the detector of Fig. 1 to follow the curvature of the spine in lateral imaging, per the density measuring view of Fig. 10, according to a second embodiment;

Fig. 14 is a detailed view in elevation of the detector of Fig. 1 showing the motion of the detector with respect to the C-arm

Fig. 15 is a schematic representation of motion of the C-arm showing the increased effective angular range obtained with the C-arm by motion of the radiation source and detector with respect to the C-arm itself;

Fig. 16 is a simplified histogram of the density of pixels within a region of interest of a patient showing the concentration of pixels into four density ranges or modes of: soft tissue, hard tissue (bone), osteophytes, and metal;

Fig. 17 is a flow chart showing a method for operation of the computer of Fig. 1 to identify and mask pixels attributable to osteophytes;

Fig. 18 is a schematic view in elevation of the C-arm and patient showing "external volumes" within a body surrounding a contrasting compact structure of interest, said external volumes not within the field-of-view fan beam but nevertheless attenuating the fan beam at some C-arm angles; and

Fig. 19 is a block diagram of the components of the present invention showing the components employed for computed tomography.

Detebf Description of the Preferred Embodiment

## Detailed Description of the Preferred Embodiment

### I. General Features of the Densitometer

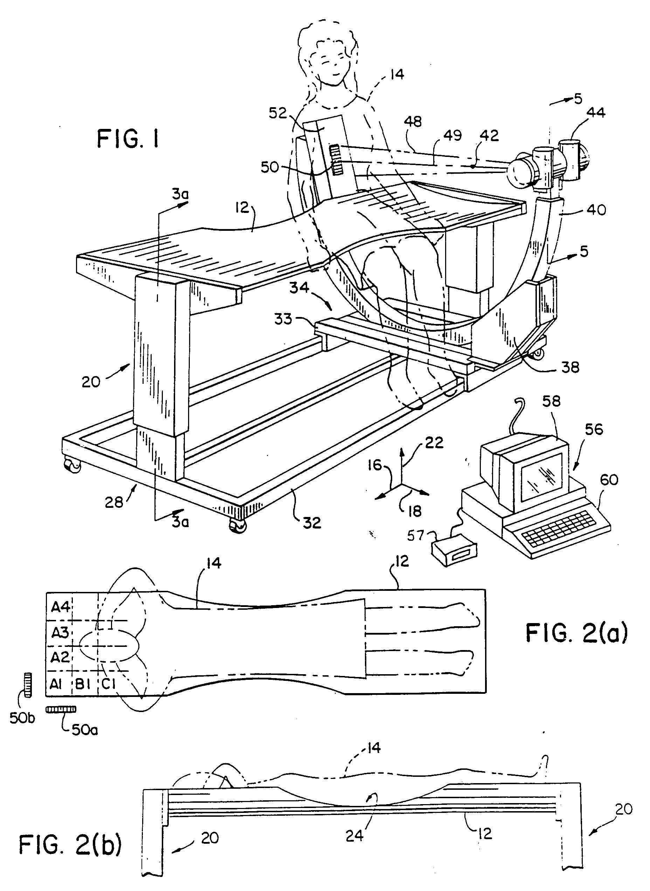

Referring to Fig. 1, a bone densitometer constructed according to the present invention includes a table 12 for supporting a patient 14 in a sitting position prior to or after an examination (as shown) or in a supine position along the table's longitudinal axis 16 during an examination. The table 12 is constructed of epoxy impregnated carbon fiber laminated over a foamed plastic core. This combination of materials is extremely light, and thus generally radioluscent, and stiff. Further, the attenuation is extremely uniform so as to prevent the introduction of artifacts into the radiographic images. The table 12 has a generally linear cross-section along the longitudinal axis 16 and an upwardly concave cross-section along a transverse axis 18 perpendicular to the longitudinal axis 16. Thus, the table 12 is a trough-shaped sheet whose transverse curvature provides additional resistance to longitudinal bending.

Support pillars 20 hold either longitudinal end of the table 12. The support pillars 20 are separated by a distance greater than the typical height of the patients to be examined so that the support pillars 20 do not obstruct the scanning process nor attenuate the measuring radiation. The longitudinal stiffness of the table 12 allows it to bridge the distance between the pillars 20 as an unsupported horizontal span thereby eliminating additional radiation attenuating structure.

In one embodiment shown in Fig. 2(b) the transverse width of the table 12 varies along its longitudinal extent being widest near the support pillars 20, and thus near the head and feet of the patient 14 when the patient 14 is in the supine position on the table 12, and narrowest at the mid-portion of the table 12 corresponding generally to the area of the patient's vertebrae when the patient is in the supine position. This narrowing of the table 12 is in the form of two rounded notches 24 extending inward toward the center of the table from either transverse edge and imparting to the table an hourglass shape as viewed along a vertical axis 22 perpendicular to the longitudinal and transverse axes 16 and 18 respectively.

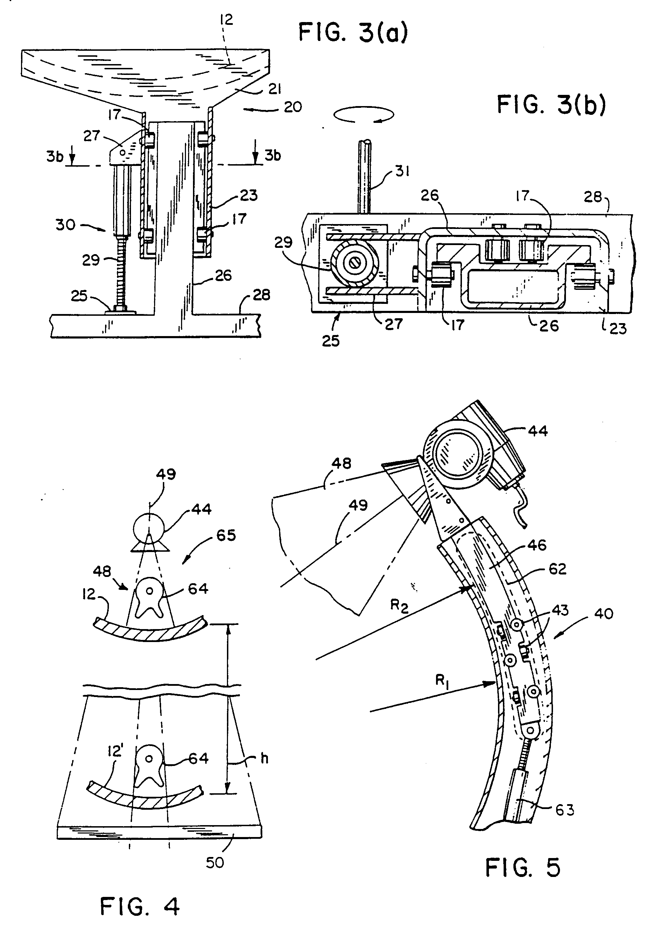

Referring now to Figs. 1, 3(a) and 3(b), support pillars 20 extend vertically downward around upward extending posts 26, the latter which are attached, at their bottom ends, to a bed 28 supporting the densitometer 10. The support pillars 20 each include a horizontal architrave 21, extending the width of the table 12 and attached to a respective end of the table 12, and vertical channel shaped casing 23 surrounding the posts 26 to vertically slide in engagement with the posts 26 guided by a set of rollers 17 attached to the casing 23. The casings 23, and hence the support pillars 20, may be positioned vertically as driven by actuators 30 each comprising a nut 27 attached to an outer casing wall and a lead screw 29 received at one end by the nut 27 and at the other end by a right angled drive 25 attached beneath the nut to the bed 28. A common drive shaft 31 connects each right angled drive 25 to a single stepper motor (not shown) so that rotation of the drive shaft 31 turns the right angled drives 25 and hence the lead screws 29 in tandem so as to raise and lower the table 12 on pillars 20 while maintaining the table's horizontal attitude. The number of steps made by the stepper motor is simply related to the change in table height.

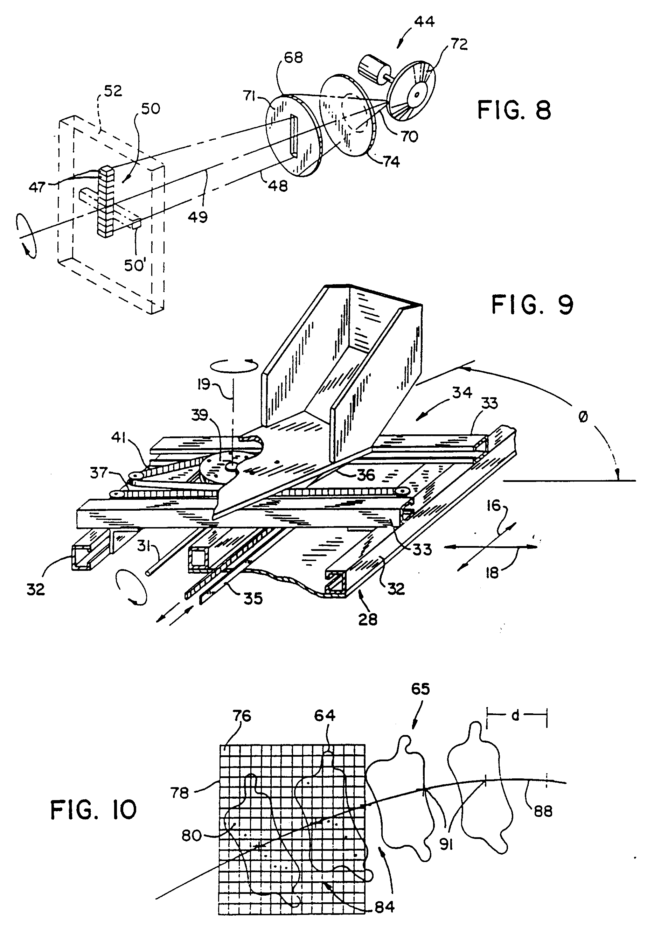

Referring to Figs. 1 and 9, the bed 28 includes two longitudinal rails 32 which form a track for supporting a transversely extending gantry pallet 34, and which allow the gantry pallet 34 to be positioned longitudinally along substantially the entire length of the densitometer 10 (as indicated by longitudinal axis 16).

The gantry pallet 34 includes transverse rails 33 carried by rollers (not visible) fitting within the rails 32 and motivated by a stepper motor driven flexible belt 35. Riding on the rails 33 of the gantry pallet 34 is a slider 36 moved transversely by stepper motor driven belt 37. The slider 36 supports a turntable 39 having a vertically oriented axis of rotation 19 and rotated by mean of stepper motor driven belt 41. As before, the stepper motors driving belts 35, 37 and 47 allow a determination of the precise movement of their respective components through a tallying of the steps taken, as will be understood to those of ordinary skill in the art.

The turntable 39 supports a C-arm collar 38. Collar 38 is generally arcuate to enclose and slidably hold a C-arm 40 such that the ends of the C-arm may rotate about an isocenter 42 as the body of the C-arm 40 slides through the collar 38. The C-arm 40 is constructed as described in U.S. Patent 4,955,046 to Aldona A. Siczek and Bernard W. Siczek entitled: "C-Arm for X-ray Diagnostic Examination". The C-arm 40 is motorized, as is understood in the art, to allow remote control over the positioning of the C-arm 40 in collar 38. The radiation source 44, which includes an x-ray tube together with filter and collimator as will be described in detail below, is mounted at one end of the C-arm 40 via a support beam 46 and is oriented to direct a polychromatic x-ray fan beam 48 along beam axis 49 generally towards the isocenter 42 to a linear detector array 50 affixed to a stop plate 52 and mounted to the opposing end of the C-arm 40.

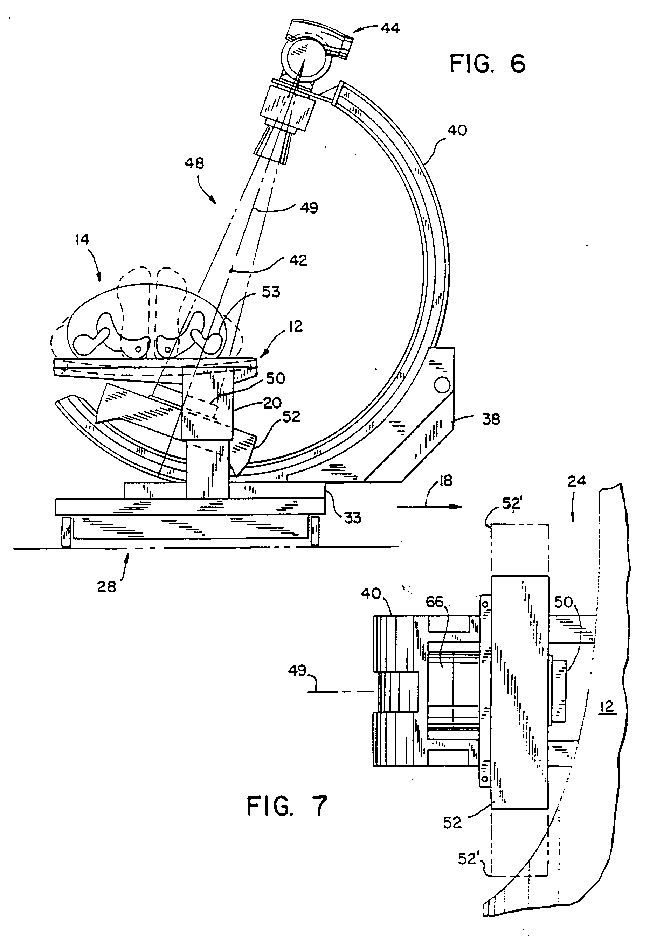

Together, motion of the pallet 34 and slider 36 permit a scanning by the detector 50 and radiation source 44 of the densitometer 10, whereas the motion of the turntable 39 allows for control of the angle of the beam axis 49 with respect to the patient 14, as will be described. The motion of the slider 36 is not limited to providing a scanning motion but may be used, in conjunction with rotation of the C-arm 40 in collar 38, to provide improved imaging of specific structures in the body without disturbing the patient 14 from the supine position. Referring to Fig. 6, imaging of the femur 53 of a supine patient 14 is ideally done at an angle of approximately 20-25° from vertical. In prior art devices this typically required uncomfortable inward rotation of the leg of the patient 14. The ability, in the present invention, both to rotate the C-arm 40 and to move the slider 36 along the transverse axis 18, and thus to move the isocenter 42, permits this imaging to be done without movement of the patient 14. Specifically, the desired angle of the C-arm 40 is simply selected and the slider 36 moved so that the beam axis 49 aligns with the femur 53.

The use of a detector array 50 of individual detector elements, each element of which may be scanned to produce an attenuation reading of one ray of the fan beam 48, provides the densitometer with the ability to scan at virtually any angle of the C-arm 40 largely independent of the limitations of the movement of the pallet 34 and slider 36 to a single plane. Scanning may be accomplished by selecting a primary scanning trajectory perpendicular to the fan beam axis 49 and calculating the protection of that trajectory on the plane of motion of the pallet 34 and slider 36 to determine the proper movement of the pallet 34 and the slider 36 that will produce motion of the C-arm 40 associated with that trajectory. As will be described below, the fan beam and the detector may then be rotated about the fan beam axis 49 so that the plane of the fan beam 48 is perpendicular to the trajectory to provide scanning, by scanning of the detector elements, in a second direction perpendicular to the primary scanning trajectory.

Alternatively, motion of the table 12 up and down combined with motion of the pallet 34 and slider 36 provides a set of three orthogonal motions that can be driven, under the control of computer 56, to produce any arbitrary primary scanning trajectory and a second perpendicular scanning motion according to basic trigonometric relations well understood to those of ordinary skill in the art.

Thus the present densitometer allows scanned images to be obtained not simply along the anterior/posterior and lateral directions, but at any angle of the C-arm 40.

Each of these actions of the C-arm 40, the slider 36 and the pallet 34 may be controlled by a computer 56 having a display terminal 58 and a keyboard 60 such as are well known in the art. By providing step commands to the motors associated with the various components above described, the computer 56 may control and locate these components, for example, by adjusting band tracking the height of the table 12, through actuators 30. Similarly, the computer 56 may control the motion of the slider and gantry pallet 34 and 36, in producing a scan or in imaging the femur 53, the angular position of the C-arm 40, as well as movement of the support beam 46 as will be described. The computer 56 also turns the radiation source 44 on and off and importantly collects digitized attenuation data from the individual elements of the linear detector array 50 to generate an array of measured points (pixels) over the patient 14.

The linear detector array 50 may be a scintillation type detector, as is understood in the art, having scintillation materials which convert x-rays to visible light to be detected by photodetectors which produce a corresponding electrical signal. Each detector element 47 of the detector array 50 incorporates two side-by-side scintillators and photodetectors to measure the x-rays fluence, of the polychromatic fan beam 48, in one of two energy bands and thus to provide, during scanning, a dual energy measurement at each point in the scan. As noted above, such dual energy measurements allow the tissue of the patient 14 being measured at a given point associated with a detector element 47 to be characterized as to its composition, for example, into bone or soft tissue.

II. Operation of the Densitometer

A. Lateral and Anterior/Posterior Positioning

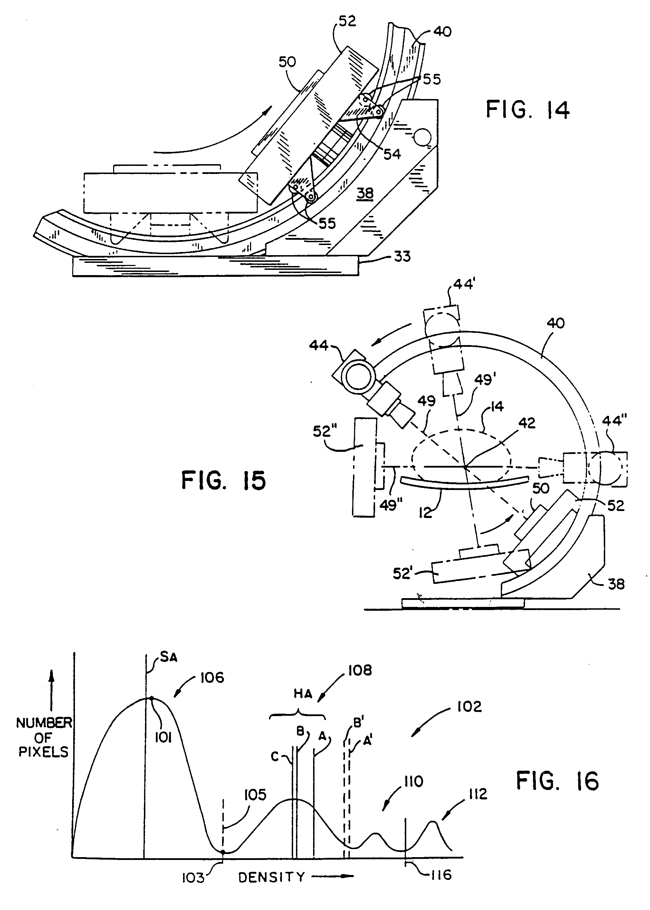

Referring now to Fig. 15, the radiation source 44 and the detector array 50 may be positioned with respect to collar 38 so that the beam axis 49 is substantially horizontal to obtain a lateral scan of the patient 14 when the patient 14 is supine on the table 12. This lateral position is indicated by phantom radiation source 44", beam axis 49" and stop plate 52". In the lateral position, the beam axis 49" will intersect the isocenter 42. Referring also to Fig. 2(b), during this lateral scan, notches 24 may provide the table with a reduced profile in the critical spinal area to eliminate attenuation of the radiation fan beam 48 by the table 12.

By sliding the C-arm 40 through the collar 38, the radiation detector may be moved to a position indicated by phantom source 44' being angularly displaced from the position of phantom source 44" by approximately 180° minus the angular extent of the collar 38. The stop plate 52 moves to the position shown by phantom stop plate 52' 180° opposed to phantom source 44'.

B. Tomographic Scanning

The angular extent of collar 38 prevents most C-arm system from realizing an angular range approaching 180° with motion of the C-arm 40 alone if the radiation source 44 and detector 50 are to be opposed about the center of rotation 42. The reason for this is the finite extent of the collar 38 along the C-arm 40 which prevents the full length of the C-arm to be used to provide rotation of the C-arm 40 and the fact that the C-arm 40 generally extends only 180° so that the radiation source 44 and the detector array 50 are opposed about the isocenter 42. As will now be described, an additional angular range may be achieved in the present invention by extending the radiation source 44 on a support beam 46 and by sliding the stop plate 52 on a track formed in the C-arm 40.

Referring now also to Fig. 5, the support beam 46 is generally arcuate with a radius $R_1$ in a first embodiment equal to the radius $R_2$ of the C-arm 40. This arcuate support beam 46 is supported by rollers 43 on a track 62 so as to telescope out of the end of the C-arm 40 and so as to move the radiation source 44 in an arc defined by radius $R_1$. The motion of the support beam 46 is accomplished by actuator 63 held within the C-arm 40 and attached between the inner most end of the support beam 46 and an internal portion of the C-arm 40 (not shown). The actuator 63 is driven by a stepper motor (not shown) so that it may operate under the control of computer 56, and so that smooth and controlled motion of the radiation source 44 may be obtained.

Referring also to Fig. 14, the stop plate 52 holding the detector 50 may be attached to a carriage 54 to slide on rollers 55 following a track formed in the inner surface of the C-arm 40. The track is positioned so that motion of the stop plate 52 is independent of position of the C-arm 40 within the collar 38, and so that the stop plate 52 may move with respect to the C-arm 40 past the collar 38 toward the position of the phantom source 44'. The motion of the stop plate 52 with respect to the C-arm 40 is controlled by the computer 56 via a stepper motor driven belt (not shown) according to methods understood to those of ordinary skill in the art.

Referring again to Fig. 15, the movement of the radiation source 44 past the end of the C-arm 40 and the motion of the stop plate 52 and detector 50 inward from the other end of the C-arm 40 allows an increase in the effective range of the angles of the beam axis 49 that may be obtained to substantially 180°.

Referring also to Fig. 5, in an alternative embodiment, the angle $R_2$ is not set equal to $R_1$. In particular, the radius $R_2$ of the C-arm 40 is the distance from the C-arm 40 to the isocenter 42. However, the radius $R_1$ of the support beam 46 is the distance between the radiation source 44 and the stop plate 52 or approximately twice that of $R_2$. Accordingly, with extension of the support beam 46, the radiation source 44 is moved so as to hold the beam axis 49 centered on the detector array 50 of the phantom stop plate 52' without movement of the stop plate 52'. In this embodiment, increased angular range may be obtained with movement only of the radiation source 44 with respect to the C-arm 40 but without movement of the detector 50 with respect to the C-arm 40.

It will be understood that in this latter embodiment, the extension of the support beam 46 increases the effective angular displacement of the radiation source 44 and detector array 50 at the cost of no longer maintaining isocentric motion about isocenter 42. The increase in effective angular rotation of the C-arm 40 will be equal to an angle α between the beam axis 49' of the radiation source 44' and the beam axis 49 of the radiation source 44 or approximately the length of the support beam 46 divided by $R_2$. This increase in the angular range achieved by C-arm 40 provides improved positioning flexibility for the densitometer 10.

As will be understood from this description, the same effective increase in the angular range of the C-arm 40 may be obtained by placing the detector array 50 on the support beam 46 and the radiation detector 50 on the carriage 54, Thus, for the purpose of tomographic scanning, an increased angular range may be obtained through the combined motions of the support beam 46 within the C-arm 40 and the carriage 54 shown in Fig. 15 along with appropriate motion of the table 12 to maintain the approximate position of the isocenter 42 within the patient 14.

A limited number of missing projections caused by restriction of the angular motion of the source 44 and the detector 50 on the C-arm 40 may be accommodated by estimating the necessary projection data through a variety of techniques generally understood in the art. In one such technique disclosed in U.S. patent 4,506,327 to Tam entitled *Limited Angle Imaging Using Multiple Energy Scanning*, hereby incorporated bye reference, the information from dual energy readings at the acquired projections is used to estimate the missing projections. Alternatively, the missing projections may be simply estimated to be values of zero, i.e., as providing complete attenuation.

## C. Adjustment of Table Height

Referring now to Fig. 4, when the radiation source 44 and detector array 50 are oriented on the C-arm (not shown in Fig. 4) so that the beam axis 49 is substantially vertical, e.g. for anterior/posterior imaging, the table 12 may be lowered by a distance *h* to move the patient's spine 65 closer to the detector array 50 (indicated by 12') and in particular to decrease the distance between the spine 65 and the detector array 50. This movement of the spine 65 close to the detector array 50 reduces the effective magnification of the image 51 generated by the data from the detector array 50 by decreasing the angular dispersion of the rays of the x-rays fan beam 48 that intersect the spine 65. It will be understood that this use of the less divergent rays of the fan beam 48 reduces the effects of parallax in the produced image providing for more accurate imaging as is desirable for morphological measurements. Parallax as used herein refers to the variations in magnification between portions of the imaged anatomy, e.g., the spine 65, closer to and further away from the radiation source 44 such as may cause blurring in the image of the outline of the spine 65 detrimental to the identification of fiducial points within the spine 65. Large amounts of parallax, caused by diverging rays at large angles from the beam axis 49, cause blurring in edges of the bone that are substantially parallel to the beam axis 49, reducing the ability of the radiologist to locate the position of these edges precisely.

Control of the table height with positioning of the C-arm is performed by the computer 56 which has data indicating the position of each component of the densitometer 10 and which may calculate clearances between components at various positions by simple geometric calculations based on the dimensions of the components of the densitometer 10 as will be understood to those of ordinary skill in the art.

In order to move the C-arm 40 within collar 38 for repositioning, the table 12 is returned to a position removed from the linear detector array 50 and close to the isocenter 42 so as to permit unobstructed movement of the C-arm 40 without interference between the radiation source 44 and stop plate 52 with the table 12. Thus, the table motion accommodates the competing desires of: (1) keeping the radius of the C-arm 40 to a minimum to produce a compact densitometer 10 and to provide accurate morphological images in the anterior/posterior direction and (2) the desire to allow flexible repositioning without interference with the table 12.

## D. Rotation of the Detector and Fan Beam

Referring now to Fig. 7, the stop plate 52 is mounted on a rotating coupling 66 which may be driven by a stepper motor actuator (not shown) under the control of the computer 56 to rotate the stop plate 52, and hence the linear detector array 50, about the beam axis 49 by 90°. For repositioning movement of the C-arm 40, the stop plate 52 which serves to absorb scattered radiation from the radiation source 44, is rotated so that its longest dimension is generally tangential to the curve of the C-arm 40, reducing the swept volume of the stop plate 52 during rotation of the C-arm 40, from the swept volume indicted by 52', and allowing it to clear the table 12. The C-arm 40 is preferably positioned so that the path of the stop plate 52 passes through the notches 24 of the table 12. The combination of the notches 24 and the rotation of the stop plate 52 serves to further reduce the necessary radius of the C-arm 40 allowing a more compact densitometer 10 to be constructed.

Referring now to Fig. 8, the rotation of the stop plate 52 about the beam axis 49 to position the detector array 50 in one of two orientations, indicated by 50 and 50' respectively, may be matched by the rotation of the fan beam 48 from the radiation source 44. This rotation of the fan beam 48, which aligns the fan beam 48 with the length of the linear detector array 50, is preferably performed not by rotating the entire radiation source 44 but by rotating a slot collimator 68 to follow to the rotation of the stop plate 52. The slot collimator 68 incorporates a slot 71 defining the width and thickness of the fan beam 48 and which allows a passage of only a portion of a cone beam 70 generated by an anode 72 of an x-ray tube of the radiation source 44. The distribution of fluence within the cone beam 70 is typically not uniform and therefore a correcting wedge filter 74 is placed between the anode 72 and the slot collimator 68 so that in either rotative position of the slot collimator 68, a uniform distribution of energy is found within the fan beam 48.

It will be understood that the divergence of the fan beam 48 is greater in its width than in its thickness, and therefore, it is generally desirable for the purposes of morphological imaging to orient the fan beam 48, and the scanning direction, so that a fiducial plane of the imaged anatomy, e.g. the inferior or superior margins of vertebrae 64 are perpendicular to the scanning direction and generally parallel to the plane of the fan beam. For example, if the superior and inferior margins of a vertebrae 64 within the patient 14 are to be measured, it is desirable that the scanning direction be generally along the longitudinal axis 16 of the spine 65, with the plane of the fan beam 48 extending perpendicularly to the scanning direction i.e., transversely.

Referring now to Fig. 2(a), the ability to rotate the detector array 50 and thus to change the scanning direction allows tailoring the acquisition of attenuation data during a scan to minimize the potential for mis-registration between adjacent scan lines caused by movement of the patient 14. Assuming that the likelihood of patient motion between acquisition of data increases with time, this requirement devolves to a requirement that adjacent areas of the patient be scanned at times that are closely proximate to each other. For example, for a whole body scan of a patient 14, the detector array 50 could be oriented transversely as indicated by 50(b) so as to scan lines longitudinally as indicated generally by the sequence of areas A1, B1 and C1 from the patient's head to the patient's foot. At the end of this scan, a second longitudinal row of data would be taken conforming generally to the sequence of areas A2, B2 and C2. Under this scanning procedure, however, area A1 and B1 which are closely proximate, are separated by the considerable length of time required to scan the entire length of the patient 14. Preferably then, the detector array 50 may be positioned longitudinally as indicated by 50(a) to scan in columns by the sequence of A1, A2 and A3 and then B1, B2, B3. Here, the greatest motion induced discontinuity will be between area A1 and B1, however the length of time between the acquisition of data for these areas will have been much reduced.

III. Two Angle Imaging

Referring now to Fig. 10, in an anterior/posterior scan of the patient 14, where the beam axis 49 is oriented vertically, the data of a rectilinear array 78 of pixels 76 is acquired. Each pixel 76 of the array 78 has a location corresponding to a particular path of a ray of the fan beam 48 through the patient 14 to one detector element 47 of the detector array 50 and each pixel 76 having a value related to the attenuation of that ray as it passes through the patient 14. As is understood in the art, the computer 56 stores the pixel values and their relative spatial locations so that each pixel 76 may be readily identified to the particular area of the patient 14 at which the data of the pixel 76 was collected.

According to well understood dual energy imaging techniques, the value of each pixel 76 is derived from measurements of the patient at two energy levels and thus provides information indicating the composition of the material causing that attenuation. In particular, the pixel value indicates the bone mineral content of the volume of the patient corresponding to the pixel location.

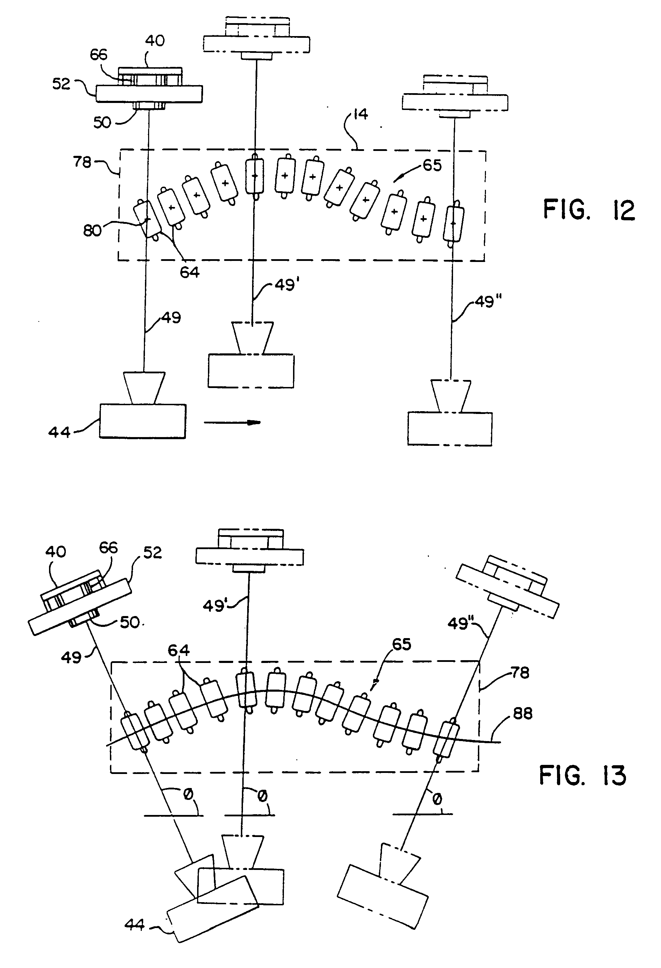

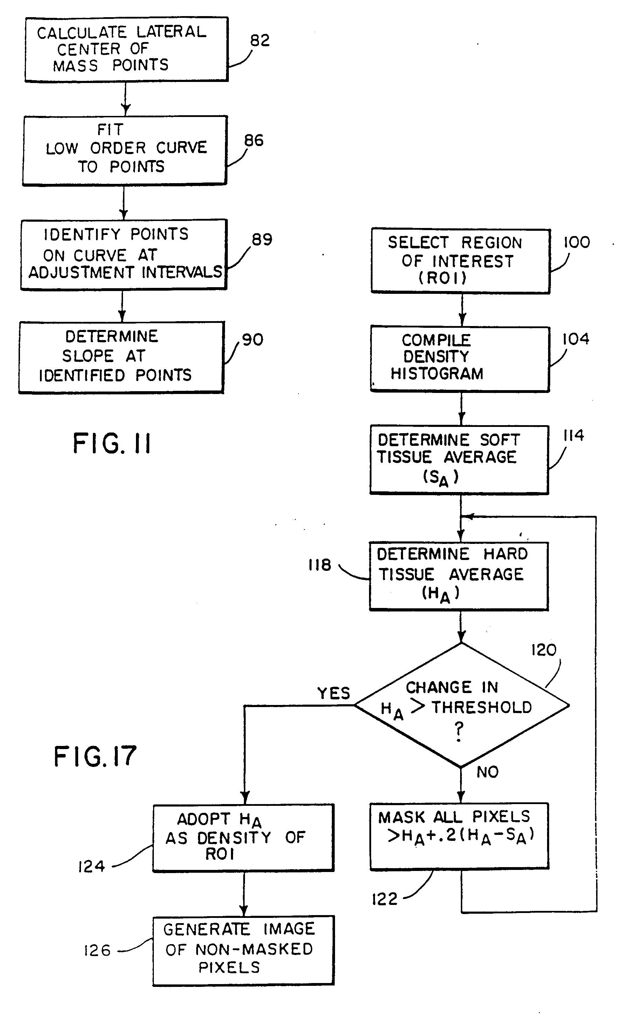

Referring to Figs. 10 and 11, after collection of the pixels 76, the computer 56 determines a center of mass (value and location) 80 for each column of the array 78, as indicated by process block 82. A column is defined relative to the principle scanning direction. For a scan of the spine 65, where movement of the detector array 50 is longitudinal, i.e., along the superior/inferior direction 16 of the patient 14, the columns of pixels 76 are transverse or perpendicular to the scanning direction.

The center of mass 80 is simply an average of the location of each pixel 76 in the column weighted by the value of the pixel 76, and thus generally indicates the center of the bone mass within that column.

The calculation of center of mass points 80 is performed by computer 56 and repeated for each column of pixel data within the array 78 to provide a general indication of the degree of lateral curvature of the patient's as evident in the anterior/posterior view. Although bone other than the spine 65 may be included within the field of view covered by the array 78, such bone, such as from the ribs, will generally cancel out in the center of mass calculation as a result of the fundamental bi-lateral symmetry of the patient 14.

In areas of low bone mass, such as intervertebral areas 84, spurious center of mass values may be obtained as a result of lack of significant bone mineral content. Accordingly, implicit in process block 82, is the elimination of calculated center of mass points 80 having a value (as opposed to location) below of predetermined threshold.

In one embodiment, illustrated in Fig. 12, the center of mass points 80 guide the positioning of the detector 50 and the radiation source 44 during a lateral scanning of the patient 14. The lateral scan is generally employed in the morphologic analysis of the vertebrae 64 and thus precise control of the magnification of the produced image is important. During the lateral scan, the center of mass points 80 of the array 78, as related to actual positions within the patient 14, are used to control the distance between the detector array 50 and the patient 14 so that the distance between the center of mass point 80 (intersected by the beam axis 49) and the detector array 50 is held substantially constant during the scan. The effect of this is that the magnification of an individual vertebra 64 in the produced image will remain substantially constant despite severe curvature or sclerosis of the spine 65 of the patient 14.

This lateral correcting movement of the detector 50 with respect to the patient 14 during the scan is provided by motion of the slider 36 (shown in Fig. 9) under control of the computer 56 as has been described. The principle motion of the scanning, in this example, is provided by motion of the pallet 34 along rails 32.

As the scan progresses, if there is no center of mass point 80 intersected by beam axis 49, the relative transverse location of the detector 50 is simply held constant. Alternatively, the position of the center of mass points 80 may be interpolated to obtain precise tracking of the detector 50 between center of mass points, such as in the intervertebral areas 84.

In this example, the anterior/posterior view is used to provide more accurate imaging in the lateral view by indicating relative lateral displacement of the imaged structure. It will recognized, however, that the above described technique is not limited to use with anterior and lateral projections but may be employed with images obtained at any two gantry angles having sufficient separation to provide the necessary third dimensions of information. Corrective motion during the second scan may be obtained by movement of various other axes of the densitometer system including vertical motion of the table 12 as has been previously described. Specifically, motion of the table may be used to provide corrected imaging of spinal curvature apparent in a lateral scan.

Referring again to Figs. 10 and 11, improved correction of the important lateral image may be obtained by fitting a low order curve 88 to the center of mass points 80 as indicated by process block 86. This low order curve 88 provides a more accurate modeling of the curvature of the spine 65 that is not perturbed by local variations in bone density within a given vertebra 64. The low curve 88 may be a simple third order polynomial fitted by least squares techniques applied by computer 56 as is well understood in the art.

After fitting of this curve 88 to the center of mass points 80, a set of scan points 91, equally spaced by d along the curve 88, may be identified according to process block 89 and the slope $\theta$ of the curve 88 at those points 91 determined by numerical differentiation according to process block 90. Typically, these scan points are separated by the pixel to pixel spacing and are shown with exaggerated spacing in Fig. 10 for clarity. These slope values $\theta_i$ where i is the index of the particular scan point 91 on the curve 88, may be used to control the angle $\theta$ at which the beam axis 49 is tipped with respect to the longitudinal axis 16 so that the beam axis 49 intersects the imaged vertebra 64 at nearly 90° to the spine axis, thus providing a sharper imaging of the laterally extending fiducial edges of the vertebra that are diagnostically significant.

Referring now to Fig. 13, the angle of the beam axis $\theta$ with respect to the principal axis of scanning 16 is controlled during the scanning along the principle axis of scanning by tilting of the detector array 50 and radiation source 44 on C-arm 40 by means of motion of turntable 39 (of Fig. 9) under the control of computer 56. Pixel data is acquired as the beam axis 49 crosses each of the predefined scan points 91.

IV. Identification of Osteophytes

Referring again to Fig. 10 certain pixels 76 of the array 78 may be identified to a region of interest (ROI) over which an average bone density value (mass per area) may be developed. Such bone density measurements are useful in evaluating the health of the bone and in tracking bone mineral loss in diseases such as osteoporosis.

Measurements of average bone density in the ROI may be distorted by the inclusion of very dense, non-bone elements such as metal pins and the like. The density of metal is much greater than that of bone and biases the average density reading upward, rendering it less accurate. Typically, the influence of metallic elements within the ROI is eliminated from the average density measurement by a simple thresholds process in which pixels having a density value greater than a fixed value are ignored in the calculation of the average.

The present invention has recognized that the average bone density value within the ROI may also be distorted by the presence of osteophytes which have a density higher than that of bone but not so high so that they can be easily separated from bone by a simple thresholds process.

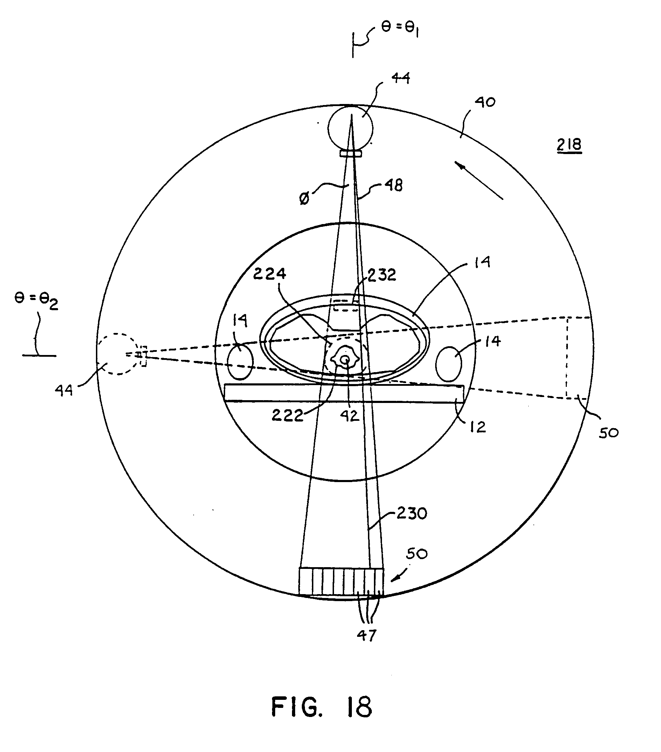

Referring to Fig. 16 and 17, once a region of interest of pixels 76 has been selected as indicated by process block 100, the particular pixel values are analyzed by the computer 56 to compile a density histogram 102 as indicated by process block 104. The density histogram plots on the horizontal axis, possible density values of each pixel 76 and on a vertical axis, the number of pixels 76 having a given density value. A typical histogram 102 will include groupings of pixels having similar density values. Such groupings will be termed "modes".

A first mode 106 includes pixels 76 of low density associated with soft tissue, and a second mode 108 includes pixels 76 of higher density 108 associated with bone, Generally, the first and second modes 106 and 108 are separated by a readily determined threshold value 105. A third mode 110 consists of pixels 76 of yet higher density of a range associated with osteopaths. This third mode 110 is shown for illustration clearly separated from the second mode 108 but in practice the separation is ill-defined. Finally, a fourth mode 112 of pixels 76 may exist being of even higher density than modes 106, 108 and 110 and associated with pixels measuring metallic pins and the like. The density of the metals is so much greater than that of the pixels of modes 106, 108; and 110 that a metal threshold 116 may be established a priori to distinguish this mode 112 from the others

As mentioned, the soft tissue mode 106 may be readily distinguished from the other modes 108-112 and isolated by identifying the first local maxima 101 and first local minima 103 after that maxima 101. This minima 103 becomes the value of the threshold 105.

At process block 114, an average density of the soft tissue pixels 106 is determined as designated $S_A$. As will be recognized to those of ordinary skill in the art, this average will simply be the center of mass of the lobe of mode 106 of the histogram 102.

At process block 118, an average density value for hard tissue ($H_A$) is determined by evaluating all those pixels not within the group 106, e.g., above threshold 105 and below the metal threshold 116. This value, $H_A$, will include certain pixels within mode 110 of the osteophytes and is indicated by line **A** on the histogram 102 of Fig. 16.

At decision block 120, the value of $H_A$ is compared to a previously computed value of $H_A$, if any. For the first iteration, there will be no previously computed value of $H_A$ and the program will proceed to process block 122 at which a threshold is established designated **A'** in Fig. 16 and being equal to the currently computed average density value of hard tissue plus 20% of the difference between the average value of the hard tissue minus the average value in density of the soft tissue or:

$$H_A + 0.2(H_A\text{-}S_A) \hspace{4cm} (0)$$

With this threshold in place, the program proceeds again to process block 118 and a new average density value for hard tissue is established, generally lower than the previous average value and indicated by line **B** of Fig. 16, and which ignores those pixels above the threshold **A'**. Typically this new value of $H_A$ will be significantly different from the previous $H_A$, i.e., by more than the predetermined threshold of decision block 120, and therefore the program loops back to process block 122 and a new threshold **B'** is computed ordinarily of a lower density value than **A'**.

This process is repeated until the newly computed value of $H_A$ converges to within the predetermined threshold of decision block 120 of the immediately preceding value of $H_A$. At this time, decision block 120 within the loop formed by process blocks 118 and 122, directs the program to process block 124 and the value of $H_A$ last computed is adopted as the bone mineral content value for the ROI. The final threshold established by line **C'** and implicit in the masking calculation of process block 122 may be used to generate an image of bone with the osteophytes removed per process block 126.

In a further embodiment, those masked pixels of group 110 may be analyzed as to their spatial locations and isolated pixels, i.e., those not having neighboring pixels within group 110 may be unmasked reflecting the rule that the osteopaths are typically of a size that spans at least two pixels of the image.

V. Limited Field-of-View Computed Tomography

A. Tomographic Geometry

Referring to Fig. 18, the radiation source 44 is mounted on the rim of the C-arm 40 to generate a diametrically oriented fan beam 48 of radiation with a narrow fan angle $\phi$. The C-arm 40 is operable to rotate through angles $\theta$ about a center of the gantry 42 within an image plane 218 with the fan beam 48 parallel to the image plane. A patient 14 is positioned at the center of the gantry 42 so that a compact structure of interest 222, such as the spine, is within the field-of-view 224 defined by the volume irradiated by the fan beam 48 at all of a plurality of gantry angles.

The fan beam 48 is received by a detector array 50 having a plurality of detector elements 47 positioned on the C-arm 40 opposite to the radiation source 44 with respect to patient 14 and the gantry center 42. Each detector element 47, distinguished by index $\alpha$, measures the intensity $I_\alpha$ of the fan beam 48 attenuated by the patient 14 along a ray 230 of the fan beam 48 at angle $\phi_\alpha$ extending from the radiation source 44 to the center of that detector element 47. The collection of intensity measurements $I_\alpha$, for all detector elements 47 at a gantry angle $\theta$ forms a projection and the collection of projections for all gantry angles $\theta$ forms a projection set.

The fan angle $\phi$ is such as to subtend the compact structure 222 at the plurality of gantry angles $\theta$ but is less than that required to subtend the entire cross section of the patient 14 in the image plane 218. This limited extent of the fan beam 48 significantly reduces the complexity and expense of the detector array 50 and the succeeding processing electronics (not shown in Fig. 18). The limited fan angle $\phi$ of the fan beam 48 also causes certain volumes elements 232 ("external volumes") of the patient 14 to contribute to a projection obtained at a first gantry angle $\theta = \theta_1$ but not to contribute to a projection at a second gantry angle $\theta = \theta_2$.

The volume subtended by the fan beam, as intercepted by the detector elements during rotation of the gantry, defines the field-of-view of the CT system.

B. Reconstruction with a Limited Width Detector

The amount of data required to reconstruct a CT image is a function of the CT system's field-of-view, the larger the field-of-view, the more data that must be collected and processed by the CT system and thus the longer the time required before an image can be reconstructed. The acquisition of additional data in each projection also increases the cost and number of the components of the CT system.

Therefore, for imaging compact structures within the body, it would be desirable to limit the field-of-view to an angle commensurate with the cross-sectional area of that compact structure. Such a reduction in field-of-view, accompanied by a reduction in the size of the fan beam, would reduce the total dose of x-rays received by the patient. In a CT machine constructed for only imaging compact structures, a reduced field-of-view would reduce the cost of the machine and provide increased image reconstruction speed as a result of the reduced amount of data required to be processed. Also, as is known in the art, smaller field of view images may be reconstructed faithfully using fewer projection angles, thereby further reducing the reconstruction times. The reduced cost of such a machine would result primarily from the reduced number of detectors and associated data handling circuitry required, and from the less powerful image reconstruction processor required to handle the amount of reduced data. Cost savings from a resulting simplified mechanical construction might also be achieved.

Unfortunately, for a CT system to accurately reconstruct images of a compact structure within an attenuating body, it is ordinarily necessary that the entire body containing the compact structure be within the CT system's field-of-view. Even when the only structure of interest is centrally located and its attenuation properties are very different than those of the rest of the section, such as the spine within an abdominal section, conventional CT methods require that substantially the entire object be within the field of view. If the body containing the compact structure extends beyond the field-of-view of the CT system, then projections at some gantry angles will include attenuation effects by volume elements of the body not present in projections at other gantry angles. For the present discussion, these volume elements present in only some projections are termed "external volumes".

In the reconstruction process, the attenuation caused by external volumes is erroneously assigned to other volume elements in the reconstructed image. This erroneous assignment produces artifacts, manifested as shading or cupping, and sometimes as streaks, in the reconstructed tomographic image and are termed "truncation artifacts".

The acquisition of two projections at two different energies of radiation from radiation source 44 can be used to eliminate the contribution of these external volumes 232 to the projections, provided that the characteristic attenuation function of the material of the external volume 232 are suitably different from those of the material of the compact structure 222. Specifically, if two projections are obtained representing the attenuation of the fan beam 48 along rays 230 by the patient 14 for two radiations energies, these projections may be used to distinguish between the attenuation caused by each of two different basis materials: one material of the external volumes 232 and one material of the compact structure 222. Thus, the attenuation of the material of the external volumes 232 and of the compact structure 222 may be determined and the effect of the former eliminated from the projections. That is, by eliminating the material of the external volumes 232 through dual energy imaging, the external volume effects are removed.

### C. Dual Imaging Components

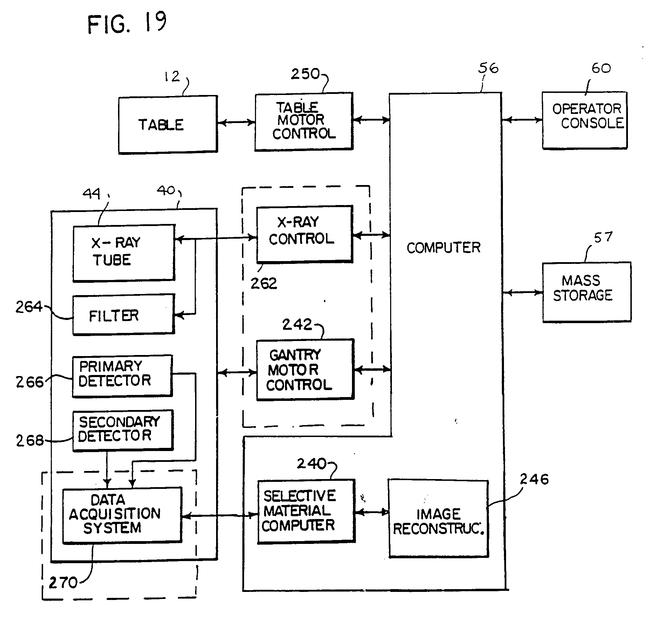

Referring now to Figs. 18 and 19, the radiation source 44 may be an unmodulated x-ray tube 44 producing a polyenergetic fan beam 48 as controlled by x-ray control 262. This fan beam 48 is filtered by stationary filter 264 to concentrate the spectral energies of the x-ray radiation into a high and low spectral lobe. Stationary filter 264 is constructed of a material exhibiting absorption predominantly in frequencies or energy between the two spectral lobes. A detector 50(c) is comprised of a primary and secondary integrating type detector 266 and 268 arranged so that the fan beam 48, after passing through the patient 14, passes first through primary detector 266 and then after exiting the primary detector, passes through the secondary detector 268. Each detector 266 and 268 is a gaseous ionization detector filled with an appropriate high atomic number gas such as xenon or a scintillation detector. Relatively lower energy x-ray photons will give up most of their energy in the primary detector 266 and be recorded as the low energy signal $I_{\alpha1}$ for that ray 230 in fan beam 48. These lower energy x-ray have a high probability of interacting in the short distance occupied by the primary detector 266 because the attenuation of the detector is higher at the lower energies. The higher energy photons will give up proportionally more of their energy in the secondary detector 268 and thereby produce the higher energy signal $I_{\alpha2}$. These two signals are collected by a data acquisition system 270 and used to produce selective material projections by circuit 240 using the polychromatic techniques described above, and reconstructed into an image as before.

The detector, alternatively may be a scintillating crystal type detector, coupled to a photomultiplier tube, or alternatively a proportional counter using xenon or other high atomic weight gas such as is well understood in the art. With either such detector, the energy level of the received radiation of the fan beam 48 is measured by a pulse height analyzer 238 which measures the energy deposited by each quantum of radiation, either pulses of light detected by the photodetector in the crystal-type detector 50(a) or pulses of charge produced by the proportional counter 50(a). The pulse height analyzer 238 characterizes each pulse, by its height, as either high or low energy. The counts of high and low energy pulses for a fixed period of time become the measures $I_{\alpha1}$ and $I_{\alpha2}$ respectively. The data for each detector element 47(a) is processed by selective material computation circuit 240 (implemented by computer 56) which performs the calculations described above (e.g. equation 4), to produce a projection set containing attenuation information for the compact structure 222 only.

Alternatively, the dual energies of radiation may be produced by switching the operating voltage of the x-ray tube 44 as is well understood in the art. Synchronously with the switching of the voltage on the x-ray tube 44, one of two filter materials of filter wheel (not shown) may be rotated into the path of the fan beam 48 on a rotating filter wheel, prior to the beam intercepting the patient 14. The filter materials serve to limit the bandwidth of the polyenergetic radiation from the x-ray tube 44 for each voltage. The filter wheel and the x-ray tube can be controlled by x-ray control 262.

In this case the detector 50 may be a single integrating type detector employing either a scintillating crystal type detector or a gaseous ionization type detector coupled to an electrical integrator may be used to produce the intensity signal, and the integrated signal for each energy level may be sampled synchronously with the switching of the bias voltage of the x-ray tube 44 and the rotation of the filter wheel 274, by data acquisition system 260 to produce the two intensity measurements $I_{\alpha1}$ and $I_{\alpha2}$ used by the selective material computation circuit 240 employing the polyenergetic corrections technique previously described (e.g. Equation (6).

After passing through the patient 14, the radiation is received by a detector array 50 comprised of a number of detector elements 47 which together receive and detect radiation along each ray 230 of the fan beam 48 to produce separate signals $I_{\alpha1}$ and $I_{\alpha2}$ for each detector element $\alpha$ and for each energy of radiation.

The control system of a CT imaging system suitable for use with the present invention has gantry motor controller 242 which controls the rotational speed and position of the C-arm 40 and provides information to computer 56 regarding gantry position, and image reconstructor 246 (implemented in computer 56) which receives corrected attenuation data from the selective material computation circuit 240 and performs high speed image reconstruction according to methods known in the art. Image reconstructor 240 is typically an array processor in a large field-of-view CT machine, however in the present invention, with a reduced field-of-view, the image reconstruction may be performed acceptably by routines running in general purpose computer 56.

Electric communication between the rotating C-arm 40 and the selective material computations circuit 240 is via retractable cabling.

Table 12 is controlled so that its upper surface translates across the image plane 218 and may be raised and lowered to position the compact structure 222 within the field-of-view 224 of the fan beam 48. The speed and position of table 12 with respect to the image plane 218 and field-of-view 224, is communicated to and controlled by computer 56 by means of table motor controller 250. The computer 56 receives commands and scanning parameters via operator console 58 which is generally a CRT display and keyboard which allows the user to enter parameters for the scan and to display the reconstructed image and other information from the computer 56.

A mass storage device 57 provides a means for storing operating programs for the CT imaging system, as well as image data for future reference by the user.

### D. Acquisition of the Dual Energy Data

Typically CT projection data is acquired over 360° of rotation, each projection including information on the attenuation of the radiation source for radiation at both of the radiation energies. As is known in the art, however, images may be reconstructed from projection data acquired over less than 360° of gantry rotation provided at least a minimum gantry rotation of 180° plus the fan beam angle is obtained. Image reconstruction using less than 360° of projection data can further reduce the data required to be processed by the image reconstructor 246. The weighing and reconstruction of images from a half scan data set are discussed in detail in "Optimal Short Scan Convolution Reconstruction for Fanbeam CT", Dennis L. Parker, Medical physics 9(2) March/April 1982. In the present invention, less than 180° plus the fan beam angle of projection data is acquired as a result of limitations of the C-arm geometry.

Preferably, two projection sets are acquired, one at high x-ray energy, and one at low x-ray energy, at each gantry angle $\theta$ before the C-arm 40 is moved to the next gantry angle $\theta$ in an "interleaved" manner so as to minimize problems due to possible movement of the patient 14. It will be apparent to one of ordinary skill in the art, however, that each projection set may be acquired in separate cycles of gantry rotation, the advantage to this latter method being that the x-ray tube voltage and the filter wheel 274 need not be switched back and forth as frequently or as fast.

### E. Dual Energy Monoenergetic Imaging

The distinction between radiation energy or frequency, and intensity or flux is noted.

The intensity measurement $I_{\alpha1}$ along a ray $\alpha$ of a first high energy of fan beam 48 radiation will be:

$$I_{\alpha1} = I_{01}\, e^{-(\mu_{e1}M_e + \mu_{c1}M_c)} \tag{1}$$

where $I_{01}$ is the intensity of the fan beam 48 of radiation absent the intervening patient 14; $\mu_e1$ and $\mu_{c1}$ are the known values of the <u>mass</u> attenuation coefficient ($cm^2/gm$) of the material of external volume 232 and of compact structure 222 respectively at this first radiation energy; and $M_e$ and $M_c$ are the integrated mass ($gm/cm^2$) of external volume

232 and of compact structure 222 respectively.

This equation may be simplified as follows:

$$\ln \frac{I_{01}}{I_{\alpha 1}} = \mu_{e1} M_e + \mu_{c1} M_c \qquad (2)$$

The values $\mu_{e1}$ and $\mu_{c1}$ of equation (1) are dependent on the energy of the radiation of the fan beam 48 and on the chemical compositions of the materials 232 and 222.

As is well known in the art, the values $\mu_{e1}$ and may be measured, or computed, given the chemical composition of the materials.

A second intensity measurement $I_{\alpha 2}$ along the same ray 230, at a second radiation energy, will be given by the following expression:

$$\ln \frac{I_{02}}{I_{\alpha 2}} = \mu_{e2} M_e + \mu_{c2} M_c \qquad (3)$$

where $\mu_{e3}$ and $\mu_{c2}$ are different from $\mu_{e1}$ and $\mu_{c1}$, by virtue of the different photon energy, and $I_{02}$ is the incident intensity. Again, $\mu_{e2}$ and $\mu_{c1}$ may be measured or computed.

Equations 2 and 3 are two independent equations with two unknowns, $M_e$ and $M_c$, and may be solved simultaneously to provide values for $M_e$ and $M_c$ For example,

$$M_c = \frac{\mu_{e1} L_2 - \mu_{e2} L_1}{\mu_{e1}\mu_{c2} - \mu_{e2}\mu_{c1}} \qquad (4)$$

where

$$L_1 = \ln \frac{I_{01}}{I_{\alpha 1}}$$

and

$$L_2 = \ln \frac{I_{02}}{I_{\alpha 2}} .$$

A stable solution requires that

$$\frac{\mu e2}{\mu e1} \neq \frac{\mu c}{\mu c}$$

This, in turn, results from the different energies of the two beams and from the different chemical compositions of the two materials (fundamentally, different relative contributions of photoelectric absorption and Compton scattering for the two materials).

With knowledge of $M_e$ and $M_c$ the contribution of the external volume 232 may be eliminated by substituting for the intensity measurement $I_{\alpha 1}$ the value

$$I_{01} e^{-\mu_{c1} M_c},$$

or more simply, by using the calculated value $M_c$ directly in the reconstruction algorithms as is understood in the art. The creation and measurement of two monoenergetic radiation beams will be described further below.

## F. Dual Polyenergetic Imaging

Factor imaging requires a stronger radiation sources 44, which also often entails an increase in the width of the energy spectrum of the radiation source 44 at each energy E. For such broadband radiation, equations (2) and (3) above, become more complex requiring an integration over the spectrum of the radiation source 44 as follows:

$$I_{\alpha} \int I_0(E)e^{-\{M_e\mu_e(E) + M_c\mu_c(E)\}}dE \qquad (5)$$

Such equations do not reduce to a linear function of $M_e$ and $M_c$ after the logarithm, and hence more complex non-linear techniques must be adopted to evaluate $M_e$ and $M_c$.

One such technique, termed the *closed form fit* approximates the value of $M_c$ as a polynomial function of the log measurements along ray a at a high and low energy, for example:

$$M_c = k_1L_1 + k_2L_2 + k_3L^2_1 + k_4L^2_2 + k_5L_1L_2 \qquad (6)$$

$M_e$ can similarly be computed.

It will be recognized that polynomials of different orders may be adopted instead. The coefficients of the polynomial, $k_1$ through $k_5$, are determined empirically by measuring a number of different, calibrated, superimposed thicknesses of the two materials to be imaged. Alternatively, it is known that the total measured polyenergetic attenuation can be treated as if the attenuation had been caused by two dissimilar "basis" materials. Aluminum and Lucite™ have been used as basis materials. The computed basis material composition is then used to compute $M_e$ and $M_c$. The advantage of this approach is that it is easier to build calibration objects from aluminum and Lucite™ than, for example, bone and soft tissue. The decomposition of an arbitrary material into two basis materials and further details on selective material imaging are described in the article "Generalized Image Combinations in Dual KVP Digital Radiography", by Lehmann et al. Med. Phys. 8(5), Sept/Oct 1981.

The determination of the coefficients of equation (6) is performed with a radiation source having the same spectral envelope as the radiation source 44 used with the CT apparatus. The coefficients are determined using a Least Squares fit to the empirical measurements developed with the known thicknesses of the models.

As indicated by the above discussion, the ability to distinguish between two materials 232 and 222, and thus the ability to discount the effect of one such material (232) requires a differential relative attenuation by the materials caused by photoelectric and the Compton effects. This requirement will be met by materials having substantially different average atomic numbers and is enhanced by increased difference in the two energies.

It is possible that the external volumes 232 of the patient 14 will include more than one type of material. An examination of the equations (3) and (4), however, reveals that the above described method will not unambiguously identify the thicknesses of a material in the presence of more than two material types within the patient 14. As a result, the above described method works best when the material of the compact structure 222 and the materials of the external volumes 232 have sufficiently different attenuation functions so that the variations among tissue types of the external volumes 232 are small by comparison. Examples are where the compact structure 222 is bone and the external volumes 232 are muscle, water or fat; or where the compact structure contains iodinated contrast agent and the external volumes 232 do not. These limitations are fundamental to dual energy selective material imaging and are not unique to the present use. In any case, errors resulting from the simplifying assumption of their being only two materials in patient 14, one for the compact structure 222 and one for the external volumes 232 are low enough to permit the above method to be used for the intended reduction of image artifacts.

The above description has been that of a preferred embodiment of the present invention. It will occur to those that practice the art that many modifications may be made within the scope of the invention.

## Claims

1. A method of making measurements of vertebrae of a patient's spine with a densitometer having an opposed radiation source (44) and detector (50), the source (44) and detector (50) moving to a first and second angle about the spine (65) and at each angle scanning to obtain a two dimensional array (78) of pixels (76) representing the attenuation of radiation at corresponding locations through the patient, comprising the steps of:

   positioning the source and detector at the first angle and scanning the vertebrae along a first axis to obtain the first array of pixels;
   analyzing the first array of pixels (76) to determine center of bone mass values (80) at a plurality of points along an axis of the array corresponding to the first axis;

positioning the source (44) and detector (50) at a second angle substantially perpendicular to the first angle;

scanning along the first axis of the patient to obtain the second array of pixels (76);

adjusting the position of the detector (50) with respect to the patient during the scanning at the second angle in accordance with the determined center of bone mass values (80) of the first array of pixels.

2. The method of claim 1 wherein the step of adjusting the position of the detector (50) adjusts the distance of the detector from the patient so as to render the distance between the detector (50) and the center of bone mass (80), as intersected by a line between the source and detector, substantially constant during the scanning at the second angle.

3. The method of claim 1 or claim 2 including the additional steps of:

disregarding those center of bone mass values (80) for points along the axis of the array where the center of mass values are below a predetermined threshold; and

fitting a curve to the remaining plurality of center of bone mass values and employing points on the curve as the center of bone mass values.

4. The method of claim 3 including the step of determining the slope of the curve to produce a set of slope values; and wherein the step of adjusting the position of the detector adjusts the angle of a line between the source and detector so that the intersection of the line and the curve as superimposed on the patient is at an angle so that the line and curve are substantially perpendicular at the intersection.

5. A method of identifying osteophytes in a bone density image comprising the steps of:

(a) employing a bone densitometer to obtain a plurality of pixels (76) each having a density value and a spatial location;

(b) determining a region of interest comprising selected pixels of the image;

(c) reviewing the selected pixels to create a multiple mode density histogram providing frequency of density values of pixels as a function of density value;

(d) identifying the lowest density mode to produce a soft tissue average $S_A$;

(e) identifying a second lowest density mode of pixels not in the lowest density mode to produce a hard tissue average $H_A$;

(f) establishing a threshold above the hard tissue average based on the difference between the hard tissue average and the soft tissue average;

(g) recomputing the hard tissue average ignoring pixels above the threshold;

(h) repeating steps (a) through (f) until the change in the computed hard tissue average is below a predetermined threshold; and

(i) displaying the current hard tissue average.

6. The method of any one of the preceding claims wherein the bone densitometer has a collar (38) for slidably holding a C-arm (40), the C-arm (40) having a first and a second end and movable through the collar (38) so as to rotate the first and second ends to a plurality of angles about the patient;

a radiation source (44) and a detector (50) affixed respectively to the first and second end in opposition about a center (42), the detector and radiation source providing energy attenuation measurements along a base axis therebetween at the plurality of angles;

an electronic computer (56) controlling the C-arm (40), radiation source (44) and detector (50) according to stored program.

7. The method of any one of the preceding claims wherein the radiation source (44) includes an x-ray tube for producing a substantially conical beam (70) of radiation and a slot collimator (68) for collimating the conical beam to a planar beam (48).

8. The method of claim 7 including a filter (74) interposed between the x-ray tube and the slot collimator (68) for filtering the conical beam (70) such that the flux density of radiation passing through the filter (74) is substantially symmetric about the radiation axis (49).

FIG. 1

FIG. 2(a)

FIG. 2(b)

FIG. 3(a)

FIG. 3(b)

FIG. 4

FIG. 5

18

FIG. 6

FIG. 7

FIG. 8

FIG. 9

FIG. 10

FIG. 12

FIG. 13

CALCULATE LATERAL
CENTER OF
MASS POINTS — 82

FIT
LOW ORDER CURVE — 86
TO POINTS

IDENTIFY POINTS
ON CURVE AT — 89
ADJUSTMENT INTERVALS

DETERMINE
SLOPE AT — 90
IDENTIFIED POINTS

## FIG. 11

SELECT REGION
OF INTEREST — 100
(ROI)

COMPILE
DENSITY — 104
HISTOGRAM

DETERMINE SOFT
TISSUE AVERAGE — 114
$(S_A)$

DETERMINE HARD
118 — TISSUE AVERAGE
$(H_A)$

## FIG. 17

CHANGE IN
YES ← $H_A$ > THRESHOLD — 120
?

NO

ADOPT $H_A$
AS DENSITY OF
ROI
124

124

MASK ALL PIXELS
> $H_A + .2(H_A - S_A)$
122

GENERATE IMAGE
126 — OF NON-MASKED
PIXELS

FIG. 14

FIG. 15

FIG. 16

FIG. 18

FIG. 19

## EUROPEAN SEARCH REPORT

**European Patent Office**

Application Number

EP 96 11 7335

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO 91 07131 A (M.R. MARQUARDT)<br>* page 2, line 17 - page 3, line 21 *<br>* page 7, line 29 - page 8, line 23 *<br>* page 10, line 26 - page 11, line 5 *<br>--- | 1,3,4,6 | A61B6/00 |
| A | US 4 606 062 A (Y. SAITO)<br>* column 4, line 5 - line 65 *<br>--- | 1,2,6 | |
| A | US 4 845 747 A (K. KOIKE ET AL.)<br>* column 1, line 60 - column 2, line 22 *<br>* column 2, line 52 - column 3, line 8 *<br>--- | 1,6-8 | |
| A | FR 2 260 977 A (DIGITAL INFORMATION SCIENCE CORP.)<br>* page 4, line 2 - line 20 *<br>* page 6, line 35 - page 7, line 2 *<br>--- | 1,4,6 | |
| D,P,<br>A | US 5 228 068 A (R.B. MAZESS)<br><br>* column 4, line 35 - column 5, line 14 *<br>* column 7, line 3 - line 36 *<br>* column 8, line 68 - column 9, line 51 *<br>----- | 1,3,6-8 | **TECHNICAL FIELDS SEARCHED** (Int.Cl.6)<br><br>A61B |

The present search report has been drawn up for all claims

| Place of search | Date of completion of the search | Examiner |
|---|---|---|
| THE HAGUE | 13 January 1997 | Rieb, K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)